# EUROPEAN PATENT SPECIFICATION

(11) **EP 2 635 127 B1**
(45) Date of publication and mention of the grant of the patent: **17.10.2018**
(21) Application number: 11838925.3
(22) Date of filing: 04.11.2011
(51) Int. Cl.: A61K 36/074, A61K 36/537, A61K 36/539, A61K 31/165

(54) **COMBINATION THERAPY FOR PROSTATE CANCER USING BOTANICAL COMPOSITIONS AND BICALUTAMIDE**
KOMBINATIONSTHERAPIE FÜR PROSTATAKREBS MIT PFLANZLICHEN ZUSAMMENSETZUNGEN UND BICALUTAMID
MULTITHÉRAPIE POUR LE CANCER DE LA PROSTATE UTILISANT DES COMPOSITIONS VÉGÉTALES ET DU BICALUTAMIDE

(30) Priority: 04.11.2010 US 410327 P
(43) Date of publication of application: 11.09.2013
(73) Proprietor: Genyous Biomed International, Redwood Shores, California 94065 (US)
(72) Inventor: DAO, James, Henderson Nevada 89052 (US); GAO, Allen, Chuan, Davis California 95616 (US); GERWICK, William, La Jolla California 92037 (US); GERWICK, Lena, La Jolla California 92037 (US); JORDAN, MaryAnn, Santa Barbara California 93105 (US); MIKOVITS, Judy, Oxnard California 93035 (US); OKOUNEVA, Tatiana, Santa Barbara California 93111 (US); OROUDJEV, Emin, Santa Barbara California 93111 (US); DAO, Jeffrey, San Mateo, California 94402 (US)
(74) Representative: AWA Sweden AB
(86) International application number: PCT/US2011/059471
(87) International publication number: WO 2012/061790

(56) References cited:
- US-A1- 2005 208 070
- US-A1- 2009 130 118
- US-A1- 2010 196 471
- REBEKAH A. BURICH ET AL: "Genistein combined polysaccharide enhances activity of docetaxel, bicalutamide and Src kinase inhibition in androgen-dependent and independent prostate cancer cell lines", BJU INTERNATIONAL, 1 July 2008 (2008-07-01), XP055113347, ISSN: 1464-4096, DOI: 10.1111/j.1464-410X.2008.07826.x
- CHEN ET AL.: 'Down-regulation of androgen-receptor and PSA by Phytochemicals' INTERNATIONAL JOURNAL ONCOLOGY vol. 32, 2008, pages 405 - 411, XP002660757

## Description

### TECHNICAL FIELD OF THE INVENTION

This invention relates generally to the field of compositions for treatment of cancer. More specifically, the invention provides multifunctional, multitargeted compositions of botanical extracts in combination with bicalutamide for the prevention and therapy of cancer, and specifically prostate cancer.

### BACKGROUND OF THE INVENTION

Prostate cancer is cancer that forms in tissues of the prostate (a gland in the male reproductive system found below the bladder and in front of the rectum). Prostate cancer usually occurs in older men. According to the National Cancer Institute, it is estimated that 217,730 new cases and 32,050 deaths from prostate cancer in the United States in 2010.

Most prostate cancers are slow growing; however, there are cases of aggressive prostate cancers. The cancer cells may metastasize (spread) from the prostate to other parts of the body, particularly the bones and lymph nodes. Prostate cancer may cause pain, difficulty in urinating, problems during sexual intercourse, or erectile dysfunction. Other symptoms can potentially develop during later stages of the disease.

Treatment options for prostate cancer with intent to cure are primarily surgery, radiation therapy, and proton therapy. Other treatments, such as hormonal therapy, chemotherapy, cryosurgery, and high intensity focused ultrasound (HIFU) also exist, depending on the clinical scenario and desired outcome.

The age and underlying health of the man, the extent of metastasis, appearance under the microscope, and response of the cancer to initial treatment are important in determining the outcome of the disease. The decision whether or not to treat localized prostate cancer (a tumor that is contained within the prostate) with curative intent is a patient trade -off between the expected beneficial and harmful effects in terms of patient survival and quality of life

Bicalutamide (marketed as Casodex®, Cosudex®, Calutide®, Kalumid®) is an oral nonsteroidal anti-androgen used in the treatment of prostate cancer (Schellhammer PF, Davis JW Clin Prostate Cancer. 2004 Mar;2(4):213-219) and hirsutism. (Muderris II, et al, Gynecol. Endocrinol. 16 (1): 63-66 (2002)). It was first launched in 1995 as a combination treatment (with surgical or medical castration) for advanced prostate cancer and subsequently launched as monotherapy for the treatment of earlier stages of the disease. Bicalutamide is in a class of medications called nonsteroidal antiandrogens. It works by blocking the effect of androgen (a male hormone), to stop the growth and spread of cancer cells.

Bicalutamide is marketed by AstraZeneca with the brand names Casodex® and Cosudex®. It is recommended in combination with a luteinizing hormone -releasing hormone analog or surgical castration. Bicalutamide is used with another medication (luteinizing hormone-releasing hormone [LHRH]; such as leuprolide or goserelin) to treat stage D2 metastatic prostate cancer (cancer that started in the prostate and has spread to other parts of the body), or as a monotherapy. (Schellhammer PF, et al, Urology 50 (3): 330-336 (1997)). It has also been used in clinical trials for ovarian cancer. (Levine D, et al., Cancer 110 (11): 2448-2456 (2007)) It has also been used in combination with castration. (Klotz L, et al., Clin Prostate Cancer 3 (4): 215-219 (2005)).

Bicalutamide along with the luteinizing hormone-releasing hormone may help stop the growth and spread of cancer cells but does not cure prostate cancer. Most advanced prostate cancer patients eventually become resistant to antiandrogen including bicalutamide therapy.

There is a need for enhanced prostate cancer therapy regimens that increase effectiveness of the therapy while reducing side effects and toxicity resulting from the chemotherapeutic treatment.

Compositions of botanicals comprising therapeutically effective amounts of two or more of an extract of Ganoderma lucidum, an extract of Salvia miltiorrhiza and an extract of Scutellaria barbata for prevention and therapy of cancer have been reported by Dao et al. (US Pat. App. Pub. No. 20050208070).

### SUMMARY OF THE INVENTION

The present invention relates to a botanical combination and bicalutamide for use in the treatment or reducing the severity of prostate cancer.

The compositions of botanical extracts can be used to reduce or alleviate the side affects when used with standard non-botanical chemotherapies. Side effects are reduced by inhibiting inflammatory responses, modulating immune responses, reducing oxidative stress, modulating immune responses, inhibiting viral and microbial infections, modulating cell proliferative responses or other biological responses. The compositions of the invention may also alleviate side affects of standard therapeutic agents by balancing general biological responses against perturbations in specific biological pathways due to treatment with the therapeutic agent.

The botanical composition comprises combinations of extracts of Ganoderma lucidum, Scutellaria barbata, and Salvia miltiorrhiza, and optionally, Hippophae rhamnoides (sea buckthorn).

In one embodiment disclosed herein, treatment or therapy of prostate cancer in a human is provided, the method comprising: administering an effective amount of a botanical composition that is effective for reducing androgen receptor protein expression; and administering concurrently an effective amount of a compound having anti-androgen activity, wherein the concurrent administration of the compound and the botanical composition achieves more effective therapy than either agent alone. Botanical compositions comprising non-alcoholic organic extracts of Ganoderma lucidum, Salvia miltiorrhiza, and Scutellaria barbata in conjunction with bicalutamide therapy are used.

The present invention and other objects, features, and advantages of the present invention will become further apparent in the following Detailed Description of the Invention and the accompanying Figures and embodiments.

### BRIEF DESCRIPTION OF THE FIGURES

Figure 1 shows the effect of the combination of Ganoderma lucidum, Scutellaria barbata, and Salvia miltiorrhiza (OMN54) and bicalutamide treatment on C4-2 cells.
Figure 2 shows effect of OMN54 and bicalutamide treatment on AR expression in C4-2 cells by Western blot analysis.
Figure 3 shows the effect of combination of OMN54 and bicalutamide on clonogenic ability in C4-2 cells.
Figure 4 shows effect of combination of OMN54 and bicalutamide on clonogenic ability in C4-2 cells.
Figure 5 shows the effect of combination of OMN54 and bicalutamide on LNCaP cell growth. LNCaP cells were treated with OMN54 and bicalutamide either alone or combination for 48 hr.

### DETAILED DESCRIPTION OF THE INVENTION

### Definitions

The term "plant" as used herein refers to seeds, leaves, stems, flowers, roots, berries, bark, or any other plant parts that are useful for the purposes described. For certain uses, it is preferred that the underground portion of the plant, such as the root and rhizoma, be utilized. The leaves, stems, seeds, flowers, berries, bark, or other plant parts, also have medicinal effects and can be used for preparing tea and other beverages, cream, and in food preparation.

The term "treatment" or "treating" or 'therapy" as used herein, for purposes of the specification and claims, includes preventing, inhibiting, curing, or alleviating.

By the term "administering," it is meant that the compositions are delivered to the host in such a manner that it can achieve the desired purpose. As mentioned the compositions can be administered by an effective route, such as orally, topically, rectally, etc.

"Synergism" may be measured by combination index (CI). The combination index method was described by Chou and Talalay. (Chou, T.-C. The median-effect principle and the combination index for quantitation of synergism and antagonism, p. 61-102. In T.-C. Chou and D. C. Rideout (ed.), Synergism and antagonism in chemotherapy. Academic Press, San Diego, Calif. (1991); Chou, T.-C, and P. Talalay. Quantitative analysis of dose-effect relationships: the combined effects of multiple drugs on enzyme inhibitors. Adv. Enzyme Regul. 22:27-55 (1984)). A CI value of 0.90 or less is considered synergistic, with values of 0.85 being moderately synergistic and values below 0.75 being significantly synergistic. CI values of 0.90 to 1.10 are considered to be merely additive and higher values are antagonistic.

**Table 1. Synergism/antagonism as a function of CI values**

| **Combination Index (CI) Value** | **Interpretation** |
|---|---|
| >10 | Very strong antagonism |
| 3.3 - 10 | Strong antagonism |
| 1.45 - 3.3 | Antagonism |
| 1.2 - 1.45 | Moderate antagonism |
| 1.1 - 1.2 | Slight antagonism |
| 0.9 - 1.1 | Additive |
| 0.85 - 0.9 | Slight synergism |
| 0.7 - 0.85 | Moderate synergism |
| 0.3 - 0.7 | Synergism |
| 0.1 - 0.3 | Strong synergism |
| < 0.1 | Very strong synergism |

It is noted that determination of synergy may be affected by biological variability, dosage, experimental conditions (temperature, pH, oxygen tension, etc.), treatment schedule and combination ratio.

A botanical composition drug include: bio-availability and minimal toxicity. Preferably the drug can be administered orally. The botanical composition provides a combination of multiple therapeutic functions to act simultaneously and synergistically on multiple biological targets. The botanical composition comprises low doses of individual therapeutic ingredients to minimize disruption of physiological homeostasis and development of drug resistance. Typically, a history of therapeutic efficacy and safety is considered in selecting nature-derived active ingredients.

### Cancer:

Several cellular pathways have been implicated in cancer. Apoptotic pathway, mitogen-activated protein kinase (MAPK) Signaling Pathway, cell signaling via the phosphoinositide 3-kinase (PI3K) pathway, Signal transducers and activators of transcription (STAT) signaling pathway, p53 signaling pathway, Wnt signaling pathway, cyclooxygenase (COX) enzymes have all been known to contribute to several steps involved in tumor formation, such as neoplastic transformation, metastasis, and angiogenesis. Also, growth factors, oncogenes such as Ras mutations, tumor suppressor genes, androgen and estrogen receptors, co-activators & repressors, and numerous others pathways have been shown to affect tumor formation and carcinogenesis.

A botanical drug suitable for cancer would include functions to affect one or more of these pathways as well as functions generally related to alleviation of cancer conditions such as anti-inflammation, immune system modulation, anti-angiogenic, anti-metastatic, and the like.

A botanical drug suitable for a particular type of cancer will possess functionalities that also are directed to pathways unique to a type of cancer. For example, botanical drug suitable for the treatment of prostate cancer may include functionalities related to androgen receptors.
Preferably, the botanical drug is administered in combination with standard chemotherapy regimens to achi

Prostate cancer is a complex disease. A number of biological pathways have been implicated in prostate cancer development: growth factor activity, cell death (apoptosis), oncogenesis, tumor suppression, cell cycle modulation, cell surface modulation, androgen receptors, co-activators & repressors. Several conditions are associated with prostate disease: Benign prostate hyperplasia (BPH), prostatitis, prostatic intraepithelial neoplasia (PIN).

Benign prostatic hyperplasia (BPH) refers to the increase in size of the prostate in middle-aged and elderly men. BPH is characterized by hyperplasia of prostatic stromal and epithelial cells, resulting in the formation of large, fairly discrete nodules in the periurethral region of the prostate. Although prostate specific antigen levels may be elevated in these patients, because of increased organ volume and inflammation due to urinary tract infections, BPH is not considered to be a premalignant lesion.

Alpha blockers (a 1 -adrenergic receptor antagonists) provide symptomatic relief of BPH symptoms. When 5a-reductase inhibitors are used together with alpha blockers a reduction of BPH progression to acute urinary retention and surgery has been noted in patients with enlarged prostates. (Kaplan SA, McConnell JD, Roehrborn CG, et al (2006). Combination therapy with doxazosin and finasteride for benign prostatic hyperplasia in patients with lower urinary tract symptoms and a baseline total prostate volume of 25 ml or greater. J Urol 175(1): 217-20.)

Several botanicals show effectiveness in treating BPH and can be used as components of a botanical formulation. Serenoa repens (saw palmetto) fruit extracts are alleviating mild-to-moderate BPH symptoms with comparable efficacy to finasteride. (Wilt TJ, Ishani A, MacDonald R, (2002). Serenoa repens for benign prostatic hyperplasia. Cochrane Database Syst Rev 2002 (3), CD001423) Other botanicals effective in treating BPH include beta-sitosterol from Hypoxis rooperi (African star grass), pygeum (extracted from the bark of Prunus africana), Cucurbita pepo (pumpkin) seed and Urtica dioica (stinging nettle) root. (Wilt TJ, Ishani A, Rutks I, MacDonald R (2000) Phytotherapy for benign prostatic hyperplasia Public Health Nutr 3(4A):459-72). One double-blind trial has also supported the efficacy of rye flower pollen. (Buck AC, Cox R, Rees RWM, et al. (1990) Treatment of outflow tract obstruction due to benign prostatic hyperplasia with the pollen extract, Cernilton. A double-blind placebo-controlled study Br. J. Urol. 66:398-404).

Prostate cancer is classified as an adenocarcinoma, or glandular cancer, that begins when normal semen-secreting prostate gland cells mutate into cancer cells. Initially, small clumps of cancer cells remain confined to otherwise normal prostate glands, a condition known as carcinoma in situ or prostatic intraepithelial neoplasia (PIN). Although there is no clear evidence that PIN is a cancer precursor, it is closely associated with cancer.

Prostate specific antigen (PSA) is a 34 kD glycoprotein manufactured almost exclusively by the prostate gland. Also known as kallikrein III, PSA is a serine protease. (Lilja H. (Nov 2003). "Biology of Prostate-Specific Antigen". Urology 62 ((5 Suppl 1)): 27-33).

PSA is often elevated in the presence of prostate cancer and in other non-malignant prostate disorders such as BPH. A blood test to measure PSA is the most effective test currently available for the early detection of prostate cancer. Higher than normal levels of PSA are associated with both localized and metastatic prostate cancer (CaP). However, PSA levels can change for many reasons other than cancer. Two common causes of high PSA levels in the absence of cancer are enlargement of the prostate (benign prostatic hypertrophy (BPH)) and infection in the prostate (prostatitis).

Thus, PSA is not a perfect test. Some men with prostate cancer do not have an elevated PSA, and most men with an elevated PSA do not have prostate cancer. Short of biopsy, no noninvasive tests provide a clear diagnosis of prostate cancer.

### Botanical compositions for prostate cancer therapy

A botanical formulation presents an optimal first line therapy when high PSA levels are detected, botanical compositions, such as those disclosed herein, have very low toxicity and yet are effective against prostate cancer.

The botanical composition was designed following demonstration of a number of desirable functions among the ingredients and in the assembled composition. A number of therapeutically active chemical entities are present in Ganoderma lucidum (#9), Scutellaria barbata (#15), and Salvia miltiorrhiza (#14): ganoderic acid H, crytotanshinone, tanshinone IIA, scutellarin tetramethyl ether, scutellarin, apigenin and wogonin.

A number of chemical entities are present in the botanical composition: adenosine, ganoderic acid A, oleic acid, tanshinone IIA, scutellarin, apigenin, luteolin, and wogonin. Each of these chemical entities is known to demonstrate one or more of anti-viral, anti-inflammatory, immune modulatory, anti-angiogenic and anti-cancer/metastatic functions.

An exemplary combination of Ganoderma lucidum (#9), Scutellaria barbata (#15), and Salvia miltiorrhiza (#14) was designated OMN54, based on the synergism displayed by certain combinations of extracts of the three botanicals when each botanical is between 1% w/w and
organic medium, such as alcohol, and non-alcoholic media including ester, lipid and the like. In a preferred embodiment the extracts were made in ethyl acetate medium.

Significant synergism was expressed by botanical compositions comprised of the following three organic extracts at the specified amounts (w/w):
(i) Ganoderma lucidum at 33-50% w/w. More specifically the Ganoderma lucidum extract is selected from 33%, 35%, 40%, 42%, 44%, 45%, 46%, 46.5%, 47%, 47.5%, 48%, 48.5%, 49%, 49.5% and 50%.
(ii) Scutellaria barbata at 33-50%) w/w. More specifically the Scutellaria barbata extract is selected from 33%, 35%, 40%, 42%, 44%, 45%, 46%, 46.5%, 47%, 47.5%, 48%, 48.5%, 49%, 49.5% and 50%.
(iii) Salvia miltiorrhiza at 1-10% w/w. More specifically the Salvia miltiorrhiza extract is selected from 1%, 1.5%, 2%, 2.5%, 3%, 3.5%, 4%, 4.5%, 5%, 5.5%, 6%, 6.5%, 7%, 7.5%, 8%, 8.5%, 9%, 9.5% and 10%.

In vitro studies have indicated that OMN54 did not modulate pro-inflammatory proteins in unstimulated PBMC (suggesting safety for long-term use). In the presence of inflammatory stimulus (PHA mitogen), OMN54 suppressed inflammatory signaling (significant antiinflammatory activity). The effect of OMN54 in stimulated and unstimulated cells was observed for a number of proteins comprising cytokines, chemokines and growth factors.

The human prostate cancer cell line, LNCaP, is androgen sensitive and PSA positive. OMN54 was tested for activity on LNCaP cell line, on the expression of specific genes associated with prostate cancer. High levels of Prostate Specific Antigen (PSA) are associated with prostate cancer. The significant suppression of the PSA transcript by OMN54 is considered beneficial in the treatment of prostate cancer.

OMN54 displays profound antiproliferative effect on prostate cancer cells, inducing the apoptosis of both androgen receptor (AR)-positive (LNCaP) and AR-negative (DU-145) prostate cancer cell lines. OMN54 also displays NF-kappa B inhibition. The anti-inflammatory and antiproliferative functions displayed by the botanical composition also are effective against BPH. Thus, following detection of high PSA levels and prior to invasive tests for diagnosis of cancer, a low toxicity botanical formulation which is effective against both BPH and neoplastic states like prostate cancer and PIN, provides a promising regimen for first intervention.

Other botanicals effective against prostate cancer include capsaicin, found in red peppers, which has a profound anti-proliferative effect on human prostate cancer cells in culture and in mouse xenografts. (Mori A et al. Cancer Res. 2006 Mar 15;66(6):3222-9). Capsaicin down-regulates PSA expression by direct inhibition of PSA transcription mediated by inhibition of NF -kappa B activation by preventing its nuclear migration. (Id.)

A 2006 study of green tea derivatives demonstrated prostate cancer prevention in patients at high risk for the disease. (Bettuzzi S, Brausi M, Rizzi F, Castagnetti G, Peracchia G, Corti A (2006). "Chemoprevention of human prostate cancer by oral administration of green tea catechins in volunteers with high-grade prostate intraepithelial neoplasia: a preliminary report from a one-year proof-of-principle study". Cancer Res 66 (2): 1234-40). A phytochemical called di-indolyl-methane found in cruciferous vegetables is suspected of having anti-androgenic and immune modulating properties.

Botanicals are a valuable resource for the discovery and development of novel, naturally derived agents to treat human disease. Botanical extracts usually comprise multiple molecules and possess multiple functions useful in the treatment and prevention of disease. Botanical extracts also can function to maintain normal tissue homeostasis by affecting multiple biological pathways such as the inflammatory pathway, the immune response pathway and the oxidative stress response pathway. As a result, botanical extracts can alleviate the harmful side effects of many therapeutic agents used to treat multiple disease targets.

Botanicals have been demonstrated to be a successful source of anticancer compositions. Examples include Gynostemma pentaphyllum extract, Camellia sinensis (green tea) and
Crataegus pinnatifida (hawthorn berries) and a method of making the same are the subject of U.S. Pat. Nos. 5,910,308 and 6,168,795. Some drugs, derived from plants that are currently used in cancer therapy were designed to perturb microtubule shortening (depolymerization) or lengthening (polymerization), such as paclitaxel, docetaxel, etoposide, vincristine, vinblastine, and vinorelbine (Compton, D. A., et al., (1999) Science 286:913-914). They share a common mechanism of action of binding to tubulin, the molecule of which microtubules are composed. (Compton, D. A., et al, (1999) Science 286:913-914). At least six plant-derived anticancer agents have received FDA approval (e.g., taxol, vinblastine, vincristine, topotecan, etoposide, teniposide). Other agents are being evaluated in clinical trials (e.g., camptothecin, 9AC, and irinotecan). Botanical extracts for the treatment cancer are described in U. S. Pat. Application Publications 20050214394 A1, 20050208070 A1 and 20050196409 A1.

The present invention provides novel compositions comprising botanical extracts to treat human diseases that are associated with multiple biological pathways in their pathologies. The compositions of the invention are comprised of botanical extracts which work synergistically to modulate multiple biological pathways including but not limited to inflammatory responses, immune responses, oxidative responses, viral and microbial infections, and cell proliferative responses. These botanical extracts are Ganoderma lucidum, Scutellaria barbata and Salvia miltiorrhiza.

Described are:
(i) Ganoderma lucidum (Reishi): Ganoderma lucidum was praised for its effect of increasing memory and preventing forgetfulness in old age reported in Shen Nong Ben Cao Jing vol. 1 as early as 456-536 AD. Research on mice using orally or topically administered Ganoderma lucidum suggests that Ganoderma lucidum has anti-inflammatory activity. (Stavinoha, W., et al., (1995). Study of the anti-inflammatory efficacy of Ganoderma lucidum. In B.-K. Kim, & Y.S. Kim (Eds.), Recent Advances in Ganoderma lucidum research (pp. 3-7). Seoul Korea: The Pharmaceutical Society of Korea).
   Applications of Ganoderma for (1) chemoprophylaxis of cancer in individuals at high risk for developing cancer (2) adjuvant use in the prevention of metastasis or recurrence of cancer (3) palliation of cancer related cachexia and pain and (4) adjunctive use with concurrent chemotherapy to reduce side-effects, maintain leukocyte counts and allow a more optimal dosing of chemo or radio therapeutics has been suggested (Chang, R. (1994) Effective Dose of Ganoderma in Humans; Proceedings of Contributed Symposium 59 A, B 5th International Mycological Congress, Vancouver: pp. 117-121). Since studies of human dosage were traditional and empirical, a proper dose range of Ganoderma for therapy was calculated using this data and pharmacokinetic principals. The calculations suggested that a (1) Ganoderma dried fruit body dose of 0.5 to 1 g per day for health maintenance (2) 2 to 5 g per day if there is chronic fatigue, stress, auto immune, or other chronic health problems (3) 5 to 10 g per day for serious illness. (Chang, R. (1993) Limitations and Potential applications of Ganoderma and related fungal polyglycans in clinical ontology; First International Conference on Mushroom Biology and Mushroom products: 96).
   Described are other species of Ganoderma. For example, G. tsugae has been shown to modulate ThI/Th2 and macrophage responses in allergic murine model, and recombinantly expressed fungal immunomodulatory protein, FIP-gts, from G. tsugae inhibited telomerase activity in A549 human lung adenocarcinoma cell line (Lin, J.Y. et al, (2006) Food Chem. Toxicol.; Liao, C.H. et al., (2006) Mo. Carcinog. 45(4):220-9). Examples of other species of Ganoderma include, but are not limited to, G. applanatum, G. mongolicum, G. microsporum, G. subamboinense, G. pfeifferi, G. meredithae, G. oregonense (G oregonse), G. resinaceum, G. oerstedii, G. ungulatum, G. mirabile, G. tsugae, G. sessile, G. valesiacum, G.fornicatum, G. carnosum, G. australe, and G. boninense.
(ii) Scutellaria barbata (Skullcap): Scutellaria barbata, a traditional Chinese medicine for liver, lung and rectal tumors, has been shown to inhibit mutagenesis, DNA binding and metabolism of aflatoxin BI (AFB1) and cytochrome P450-linked aminopyrine N-demethylase (Wong B.Y. et al, (1993) Eur. J. Cancer Prev. 2(4):351-6; Wong B.Y. et al, (1992) Mutat. Res. 279(3):209-16). Scutellaria barbata is also capable of enhancing macrophage function in vitro and inhibiting tumor growth in vivo (Wong B.Y. et al., (1996) Cancer Biother. Radiopharm. I I(I):51-6).
   This herb contains vitamins C and E as well as calcium, potassium, magnesium, iron, zinc scute Ilarin, volatile oil, tannin and bitter principles. The scutellarin acts on the central nervous system. Scutellarin, an active ingredient from Scutellaria barbata has been purified by liquid chromatography (Wenzhu Zhang et al., (2003) J. of Liquid Chromatography & Related Technologies 26 (13):2133-40).
(iii) Scutellaria baicalensis: Scutellaria baicalensis has been shown to have antiproliferative and apoptotic activities against lymphocytic leukemia, lymphoma, and myeloma cell lines and possess anti-cancer activity on human malignant brain tumor cells (Kumagai, T. et al. (2006) Leuk. Res.; Scheck, A.C. et al, (2006) BMC Complement Altern. Med. 6:27).
   Scutellaria barbata should not be confused with Scutellaria baicalensis. Banzhilian, the whole plant of Scutellaria barbata, should not be confused with "scute," the common name referring to huangqin, the root of Scutellaria baicalensis. "Although both are of the same genus, Scutellaria barbata, for which the tops are used, has essential oils among the active components, while Scutellaria baicalensis relies primarily on flavonoids, particularly baicalin and baicalein. Scutellaria radix (root of Scutellaria baicalensis) and Scutellaria barbata comprise different sets of flavonoids and show different effects on proliferation of human leukemia cell line HL-60. Sonoda et al, J. Ethnopharm 91 :65-68 (2004)
   Also disclosed are other species of Scutellaria. For example, Scutellaria radix has been shown to suppress ethanol-induced caspase-11 expression and cell death in N(2)a cells, and Baicalein, a component of Scutellaria radix, leads to suppression of proliferation and induction of apoptosis in human myeloma cells (Kang, K. et al., (2005) Brain Res. Mol. Brain Res. 142(2): 139-45; Ma, Z. et al. (2005) Blood 105(8):3312-8). Examples of other species of Scutellaria include, but are not limited to, Scutellaria amabilis, Scutellaria radix, Scutellaria rehderiana, and Scutellaria lateriflora. Preferred combinations are those where the extract from a particular species acts in synergy with extracts from other botanicals in the formulation or with other therapeutic agents in the composition.
(iv) Salvia miltiorrhiza (Dan Shen): There are over 900 species of salvia and many of them have histories of medicinal uses. Dan shen is used in traditional Chinese medicine to promote blood circulation and to remove blood stasis (Bensky D., Gamble A Chinese herbal Medicine Materia Medica 1987 Eastland Press: Seattle. 384). It increases the activity of SOD in platelets, thus providing protection against pulmonary embolism and inhibition of platelet aggregation. (Wang, X. et al, (1996) Zhongguo Zhong Yao Za Zhi 21 :558-60). Salvia miltiorrhiza has been shown to lower cholesterol, reduce endothelial damage and to inhibit lipid peroxidation in hypercholesterolemic animals. This inhibition of oxidation of LDL may reduce atherosclerosis (Wu Y.J. et al, (1998) Arteriosclerosis Thromb Vase Biol 18:481-6). A Salvia miltiorrhiza constituent has been found to inhibit noradrenalin-induced contraction of the aortic strips through reduction in Ca²⁺ mobilization. This vasodilatory activity may explain the traditional use of Salvia miltiorrhiza in hypertension (Nagai M. et al., Biol Pharm Bull (1996) 19:228-32). Salvia miltiorrhiza has been shown to have a markedly superior effect to nitroglycerin, with a more persistent action and better improvement of cardiac function (Bai, Y.R. and Wang, S.Z., (1994) Zhongguo Zhong Xi Yi Jie He Za Zhi 14:24-5, 4).
   Salvia miltiorrhiza is also the top ingredient in Dan Shen Compound. Dan Shen Compound comprises four important herbs for the improvement of peripheral circulation and general wellbeing. The actions of Crataegus levigata are enhanced by the Chinese herb Salvia miltiorrhiza (Dan Shen), the Indian herb Coleus forskohlii and Valeriana officinalis. Chinese herbal medicine utilizes Salvia miltiorrhiza for women's irregularities, abdominal pain, insomnia, hives, hepatitis and mastitis.
(v) Hippophae rhamnoides (sea buckthorn): Sea buckthorn seed oil contains a high content of the two essential fatty acids, linoleic acid and a-linolenic acid, which are precursors of other polyunsaturated fatty acids such as arachidonic and eicosapentaenoic acids. The oil from the pulp/peel of seabuckthorn berries is rich in palmitoleic acid and oleic acid (Chen et al., "Chemical composition and characteristics of seabuckthorn fruit and its oil." Chem. Ind. Forest Prod. (Chinese) 10 (3), 163-175). The increase in the level of a-linolenic acid in plasma lipids showed a clear improving effect on AD symptoms (Yang et al., (2000) J. Nutr Biochem. 11(6):338-340). These effects of α-linolenic acid may have been due to both changes in the eicosanoid composition and other mechanisms independent of eicosanoid synthesis (Kelley (1992) Nutrition, 8 (3), 215-2).
   Antioxidant and immunomodulatory properties of sea buckthorn {Hippophae rhamnoides) have been demonstrated using lymphocytes as a model system. (Geetha et al. J Ethnopharmacol 2002 Mar; 79(3):373-8). The antiulcerogenic effect of a hexane extract from Hippophae rhamnoides has also been demonstrated. (Suleyman H. et al, (2001) Phytother Res 15(7):625-7). Radioprotection by an herbal preparation of Hippophae rhamnoides against whole body lethal irradiation in mice suggests free radical scavenging, acceleration of stem cell proliferation and immunostimulation properties. (Goel H.C. et al., (2002) Phytomedicine 9(I): 15-25)
(vi) Camellia sinensis (Green tea): Dried leaves from the Camellia sinensis plant is processed into three types of tea: oolong tea, black tea, and green tea. Green tea extract is a bioflavonoid-rich, potent extract which is used primarily for fighting free radicals. It has a high content of polyphenols, which are a type of bioflavonoids. In making green tea, the tea leaves are stabilized by moist or dry heat which destroys the enzyme polyphenoloxidase and thus, prevents oxidation of polyphenols. These polyphenols are the main biologically active ingredients in green tea. In preferred embodiments, the green tea is Dragon Well tea or Lung Ching tea.

The polyphenols in green tea are catechins, with multiple linked ring-like structures. Polyphenols are a form of bioflavonoids with several phenol groups. They control both taste and biological action. Catechins, a chemical group of polyphenols possessing antioxidant properties (protecting cells from free radical-mediated damage), include epigallocatechin-3 gallate (EGCG), epigallocatechin, and epicatechin-3-gallate. Recently, ECGC has been shown to be an inhibitor of urokinase (Jankun et al., (1997) Nature 387:561), and quinol-oxidase; enzymes that may be crucial for growth of tumor cells. Epigallocatechin-3 gallate (EGCG) also protects against digestive and respiratory infections.

Novel tumor inhibiting, immune boosting, inflammation reducing and anti-oxidative properties observed for compositions comprising a combination of two or more extracts of Ganoderma lucidum, Scutellaria barbata, Scutellaria baicalensis, and Salvia miltiorrhiza and, optionally, Hippophae rhamnoides (seabuckthorn) and Camellia sinensis (green tea) and the synergistic effects demonstrated by novel combinations of two or more of these extracts used in the method according to the present invention are a likely result of combinations of one or more of saponins, flavonoids, and polyphenols present in the extracts.

### Formulations of Botanical Compositions

The compositions of the present invention can be in any form which is effective, including, but not limited to dry powders, grounds, emulsions, extracts, and other conventional compositions. To extract or concentrate the effective ingredients of the compositions, typically the botanical part is contacted with a suitable solvent, such as water, alcohol, methanol, mixed solvents, or any other solvents. The choice of the solvent can be made routinely, e.g., based on the properties of the active ingredient that is to be extracted or concentrated by the solvent. Preferred active ingredients of the compositions crenulata include, but are not limited to, salidroside, tyrosol, B-sitosterol, gallic acid, pyrogallol, crenulatin, rhodionin, and/or rhodiosin. These ingredients can be extracted in the same step, e.g., using an alcoholic solvent, or they may be extracted individually, each time using a solvent which is especially effective for extracting the particular target ingredient from the plant. In certain embodiments, extraction can be performed by the following process: Milling the selected part, preferably root, to powder. The powder can be soaked in a desired solvent for an amount of time effective to extract the active agents from the compositions. The solution can be filtered and concentrated to produce a paste that contains a high concentration of the constituents extracted by the solvent. In some cases, the paste can be dried to produce a powder extract of the compositions crenulata. The content of active ingredient in the extract can be measured using HPLC, UV and other spectrometry methods.

The compositions of the present invention can be administered in any form by any effective route, including, e.g., oral, parenteral, enteral, intraperitoneal, topical, transdermal (e.g., using any standard patch), ophthalmic, nasally, local, non-oral, such as aerosol, inhalation, subcutaneous, intramuscular, buccal, sublingual, rectal, vaginal, intra-arterial, and intrathecal, etc. It can be administered alone, or in combination with any ingredient(s), active or inactive, including in a medicinal form, or as a food or beverage additive.

In preferred embodiments of the invention, the compositions are administered orally in any suitable form, including, e.g., whole plant, powdered or pulverized plant material, extract, pill, capsule, granule, tablet or a suspension.

The compositions can be combined with any pharmaceutically acceptable carrier. By the phrase, "pharmaceutically acceptable carriers," it is meant any pharmaceutical carrier, such as the standard carriers described, e.g., Remington's Pharmaceutical Science, 18th Edition, Mack Publishing company, 1990. Examples of suitable carriers are well known in the art and can include, but are not limited to, any of the standard pharmaceutical carriers such as a phosphate buffered saline solutions, phosphate buffered saline containing Polysorb 80, water, emulsions such as oil/water emulsion and various types of wetting agents. Other carriers may also include sterile solutions, tablets, coated tablets pharmaceutical and capsules. Typically such carriers contain excipients such as such as starch, milk, sugar, certain types of clay, gelatin, stearic acid or salts thereof, magnesium or calcium stearate, talc, vegetable fats or oils, gums, glycols. Such carriers can also include flavor and color additives or other ingredients. Compositions comprising such carriers are formulated by well known conventional methods. Generally excipients formulated with the compositions are suitable for oral administration and do not deleteriously react with it, or other active components.

Suitable pharmaceutically acceptable carriers include but are not limited to water, salt solutions, alcohols, gum arabic, vegetable oils, benzyl alcohols, gelatin, carbohydrates such as lactose, amylose or starch, magnesium stearate, talc, silicic acid, viscous paraffin, perfume oil, fatty acid monoglycerides and diglycerides, pentaerythritol fatty acid esters, hydroxy methylcellulose and the like. Other additives include, e.g., antioxidants and preservatives, coloring, flavoring and diluting agents, emulsifying and suspending agents, such as acacia, agar, alginic acid, sodium alginate, bentonite, carbomer, carrageenan, carboxymethylcellulose, cellulose, cholesterol, gelatin, hydroxyethyl cellulose, hydroxypropyl cellulose, hydroxypropyl methylcellulose, methylcellulose, octoxynol 9, oleyl alcohol, povidone, propylene glycol monostearate, sodium lauryl sulfate, sorbitan esters, stearyl alcohol, tragacanth, xanthan gum, and derivatives thereof, solvents, and miscellaneous ingredients such as microcrystalline cellulose, citric acid, dextrin, dextrose, liquid glucose, lactic acid, lactose, magnesium chloride, potassium metaphosphate, starch, and the like.

The botanical compositions can also be formulated with other active ingredients, such as anti-oxidants, vitamins (A, C, ascorbic acid, B's, such as BI, thiamine, B6, pyridoxine, B complex, biotin, choline, nicotinic acid, pantothenic acid, B12, cyanocobalamin, and/or B2, D, D2, D3, calciferol, E, such as tocopherol, riboflavin, K, KI, K2). Preferred compounds, include, e.g. creatine monohydrate, pyruvate, L-Carnitine, a-lipoic acid, Phytin or Phytic acid, Co Enzyme Q10, NADH, NAD, D-ribose, amino acids such as L-glutamine, Lysine, chrysin; pre-hormones such as 4-androstenedione, 5-androstenedione, 4(or 5-)androstenediol, 19-nor-4 (or 5-)-androstenedione, 19-nor-4 (or 5-)-androstenediol, Beta-ecdysterone, and 5-Methyl-7-Methoxy Isoflavone. Preferred active ingredients include, e.g., pine pollen, fructus lycii, Hippophae rhamnoides, Ligusticum, Acanthopanax, Astragalus, Ephedra, codonopsis, polygola tenuifolia Willd, Lilium, Sparganium, ginseng, panax notogiseng, Garcinia, Guggle, Grape Seed Extract or powder, and/or Ginkgo Biloba.

Other plants and herbs which can be formulated with the compositions of the present invention includes those mentioned in various text and publications, e.g., E.S. Ayensu, Medicinal Plants of West Africa, Reference Publications, Algonac, Mich. (1978); L. Boulos, Medicinal Plants of North Africa, Reference Publications Inc., Algonac, Mich. (1983); and N. C. Shah, (1982) J. Ethnopharm, 6:294-5.

A botanical formulation may comprise biologies and chemical entities, in addition to or in the place of, botanical extracts. Examples of biologies that may comprise a botanical composition include but are not limited to blood and blood products, cells, tissues and organs, gene therapy vectors, viral and bacterial vaccines, therapeutic products produced through biotechnology such as antibodies, monoclonal antibodies, and the like.

Pharmaceutically active agents that can comprise a botanical composition include, but are not limited to antioxidants, anticarcinogens, anti-inflammatory agents, hormones and hormone antagonists, anti-hypertensive agents, anti-inflammatory agents, tranquilizers, cardiotonic agents, antidepressants, corticosteroids, anti-ulcer agents, anti-allergy agents and anti-obesity agents, antibiotics, antibacterial agents, bacterial agents, and other medically useful drugs such as those identified in, e.g., Remington's Pharmaceutical Sciences, 18th Edition, Mack Publishing Company, 1990. A preferred composition of the present invention comprises, about 1%-100%, preferably about 20-70% of the botanical extract and, optionally, a pharmaceutically-acceptable excipient. Another preferred composition of the present invention comprises, about 1%-99%, preferably about 20-70% of botanical extracts, 0.1-99%, preferably 1-10% of one or more pharmaceutically active agents and, optionally, a pharmaceutically-acceptable excipient.

In some embodiments, the botanical composition comprises a chemotherapeutic agent either in a single formulation or separately administered as part of a therapeutic regimen.

According to the instant invention, it has been surprisingly observed that concurrent treatment with Bicalutamide and OMN54 results in more effective remediation of prostate cancer symptoms with significantly reduced side effects and synergistic anticancer activity.

Bicalutamide is used in monotherapy or with with another medication (luteinizing hormone-releasing hormone [LHRH]; such as leuprolide or goserelin) to treat metastatic prostate cancer (cancer that started in the prostate and has spread to other parts of the body). Bicalutamide is a nonsteroidal antiandrogen, which works by blocking the effect of androgen (a male hormone), to stop the growth and spread of cancer cells. Bicalutamide comes as a tablet to take by mouth. It is usually taken with or without food once a day, either in the morning or evening. Standard dosages of bicalutamide are dosages of 25, 50, and up to 150 mg single dose per day. For combination therapy with LHRH, bicalutamide therapy is started on the same day you begin injecting the luteinizing hormone-releasing hormone.

The botanical composition and the bicalutamide according to instant invention may be used for the administration of bicalutamide and the botanical compositions (such as OMN54) concurrently. By concurrently, it is understood that the botanical composition is administered simultaneously, or the same day, or within 24 hours of bicalutamide.

In some embodiments, the botanical composition and bicalutamide is formulated as a tablet dosage form comprising: a) a first layer comprising a tablet of bicalutamide, wherein the tablet is inlayed in the first layer with one or more other pharmaceutically acceptable excipients; and b) a second layer comprising a composition comprising extracts of Ganoderma lucidum, Scutellaria barbata, and Salvia miltiorrhiza, and optionally other pharmaceutically acceptable excipients. In some embodiments, the dosage form comprises a bilayered dosage form. In some aspects the tablet is coated with one or more enteric polymers, pharmaceutically acceptable seal coat polymers or rate controlling polymers. In some aspects, the two active ingredients are provided as a dispersion provided in a capsule, granule, mini-tablet or tablet form.

In some aspects, bicalutamide and/or the botanical extracts composition are present in an immediate release, delayed release, sustained release, extended release, controlled release or modified release form.

In other embodiments, the invention relates to a kit containing separate dosage forms for each active ingredient, for example, comprising bicalutamide provided in a tablet form and the botanical extracts provided in a capsule form. In some embodiments, the capsule and the tablet are provided in a single package (such as a blister pack).

Methods of administering the compositions are disclosed herein, e.g., to provide anti-inflammatory effects, to reduce inflammation, to provide antioxidant effects, to protect against oxidation, to provide antiproliferative effects, to provide anti-cancer effects, to promote DNA repair, to provide anti-radiation effects, to protect against radiation, and other conditions and diseases as mentioned herein.

An effective amount of the compositions are administered to such a host. Effective amounts are such amounts which are useful to achieve the desired effect, preferably a beneficial or therapeutic effect as described above. Such amount can be determined routinely, e.g., by performing a dose-response experiment in which varying doses are administered to cells, tissues, animal models (such as rats or mice in maze-testing, swimming tests, toxicity tests, memory tests as performed by standard psychological testing, etc.) to determine an effective amount in achieving an effect. Amounts are selected based on various factors, including the milieu to which the virus is administered (e.g., a patient with cancer, animal model, tissue culture cells, etc.), the site of the cells to be treated, the age, health, gender, and weight of a patient or animal to be treated, etc. Useful amounts include, 10 milligrams- 100 grams, preferably, e.g., 100 milligrams- 10 grams, 250 milligrams-2.5 grams, 1 gm, 2 gm, 3 gm, 500 milligrams- 1.25 grams, etc., per dosage of different forms of the compositions such as the botanical powder, botanical extract paste or powder, tea and beverages prepared to contain the effective ingredients of the compositions, and injections, depending upon the need of the recipients and the method of preparation.

The liquid, pharmaceutically active formulation comprises a pharmaceutically active botanical composition in a liquid diluent or carrier. The active ingredient may be dissolved or dispersed in the liquid diluent or carrier, which may be a water miscible or water immiscible medium. Examples of liquid diluents or carriers include the following three classes: (a) Water miscible carriers: Propylene Glycol, Polyethylene Glycol, Water, Solketal, Glycofurol, Dimethylisosorbide, Nonionic surface active agents; (b) Oils and Organic carriers: Fractionated Coconut Oil, Sesame Oil, Soya Bean Oil, Vegetable Oil, Liquid Paraffin, Isopropylmyristate, Triacetin; and (c) Semi-solid carriers: High molecular weight polyethylene glycols, and White soft paraffin.

In some embodiments, one or more emulsifiers or surfactants are included in the formulation. Suitable emulsifiers which can be used include one or more of fatty acids such as oleic acid, polyoxyethylene glycerol esters of fatty acids, such as Tagats; polooxylated castor oil, ethylene glycol esters, such as glycol stearate and distearate; propylene glycol esters, such as propylene glycol myristate; glyceryl esters of fatty acids, such as glyceryl stearates and monostearates; sorbitan esters, such as spans and tweens; polyglyceryl esters, such as polyglyceryl 4-oleate; fatty alcohol ethoxylates, such as Brij type emulsifiers; ethoxylated propoxylated block copolymers, such as poloxamers; polyethylene glycol esters of fatty acids, such as Labrafils, Labrafacs, and Labrasols; cremophores; glycerol monocaprylate/caprate, such as Campmul CM 10; Gelucire, Capryol, Captex, Acconon, transcutol, triacetin, and the like. In some embodiments, antioxidants and/or diluents are used in the formulation.

Compositions of the present invention comprise effective amounts of a botanical combination of extracts of Ganoderma lucidum, Scutellaria barbata, and Salvia miltiorrhiza, and optionally, Hippophae rhamnoides (sea buckthorn) that exhibit synergy, in combination with bicalutamide.

According to the invention, the botanical composition comprises effective amounts of extracts of Ganoderma lucidum, Scutellaria barbata, and Salvia miltiorrhiza. The dosage of the composition can be readily determined by one of skill in the art based on the effective concentrations of compositions shown to display the various properties described herein.

Compositions comprising different ratios of the individual extracts can similarly be determined. For example, a composition may exhibit anti-inflammatory effects at one concentration or ratios of combinations of extracts and varying degrees of cytotoxic effects at other concentrations or ratios of combinations of extracts. Any ratio of extracts of two or more of Ganoderma lucidum, Scutellaria barbata, Scutellaria baicalensis, and Salvia miltiorrhiza can be used in the compositions of the invention. It is preferred that each extract is present in the composition in equal amounts or at about 1% to about 90% of the total composition. In some embodiments of the invention, a particular extract comprises at least 1%, 1.5%, 2%, 5%, 10%, 15%, 25%, 33%, 40%, 45%, 47.5%, 48.5%, 49.5%, 50%, 60%, 66%, 75%, 90% or 98% by weight of the composition. In one embodiment the OMN54 comprises about I-3%> Salvia miltiorrhiza, and approximately equal amounts (45-50%) of Scutellaria barbata and Ganoderma lucidum.

In a further embodiment, the compositions of the present invention comprise botanical compounds that are useful in compositions to be administered in conjunction with therapeutic agents for the treatment of disease. These compositions exhibit synergistic action with the therapeutic agent based on their anti-inflammatory, antioxidant, immune modulating, antiviral, antibacterial, antiproliferative activity or any combination of activities thereof.

The compositions demonstrate antioxidant activity which prevents damage to chromosomes/genes, reduces effect of mutagens, alleviates side-effects of chemotherapeutic agents, alleviates side-effects of hormone therapeutic agents, and enhances cell repair mechanisms.

The compositions further demonstrate immune system boosting activity which facilitates elimination of (i) damaged cells or (ii) cells with damaged genes. Further, the compositions provide general benefits of improving immune condition (passive immunotherapy).

The botanical sources of the extracts are botanicals that are essentially nontoxic with a long history of usage of the individual compounds/extracts. Anti-mutagenic properties as evidenced by Ames test results (together with increased sensitivity by synergism) reduce levels of chemotherapeutic agents necessary for treatment resulting in reduced toxicity for patients.

The botanical compositions demonstrate the ability to enhanced cell cycling which could make the botanical composition of the invention a powerful adjuvant to chemotherapy (e.g., with bicalutamide), hormonal therapy, or radiation therapy by increasing effectiveness and reducing necessary dosages of chemotherapeutic agents and hormone therapeutic agents.

Quality control. IC₅₀ based compositions can be standardized based on specific activities of defined properties.

The compositions are also suited for convenient (oral) drug delivery. Compositions are extracts made with hot water, alcoholic solvents (ethanol) and non-alocoholic organic solvents (ester, lipid, ethyl acetate, etc.).

Overall the botanical compositions show mostly cytostatic effect with very weak cytotoxic effects in the compositions of the invention. Histopathology of cells treated with the compositions of the invention indicates minimal retention of dead cancer cells which enhance recovery following cancer therapy.

### EXAMPLES

Without further elaboration, it is believed that one skilled in the art can, using the preceding description, utilize the present invention to its fullest extent. The following examples are illustrative only, and not limiting of the remainder of the disclosure in any way whatsoever.

The following combinations of extracts were used throughout the examples: Ganoderma lucidum, Scutellaria barbata, optionally Scutellaria baicalensis, and Salvia miltiorrhiza extracts when combined in ratios that exhibit synergism are referred to as OMN54 in the specification.

In addition, the compositions of the invention may include, optionally, Panax Quinquefolium (Western ginseng), Camellia sinensis (green tea), and Hippophae rhamnoides (sea buckthorn).

One skilled in the art would appreciate that while the following examples are illustrative of the invention, any cell line may be used. For example, although not limiting, cells may be obtained from ATCC, Rockville, Md.

One skilled in the art would also appreciate that while the foregoing examples are illustrative of the invention, multiple prostate cancer in vivo models may be used. For example, CaP xenografts in mice may be utilized. Additionally, a Pten knockout mouse strain, in which heterozygous mice develop tumors of the uterus, prostate, thyroid, colon, and adrenal medulla, may be obtained from the Mouse Models of Human Cancers Consortium (Podsypanina K. et al., (1999) Proc. Nat'l Acad. Sci. USA 96: 1563-1568).

### Example 1 : Methods for Preparation of Botanical Extracts

The compositions of the present invention may be administered as dried herbs. Botanical preparations contain phytochemicals some of which are soluble in aqueous media while others are relatively more soluble in organic (alcohol, lipid) media. Different extraction methods were used and tested for the ability to extract effective ingredients from the herbs. Extraction methods include: Aqueous (hot water) extraction; Organic (lipid fraction) extraction; non-alcoholic organic (ethyl acetate) extraction; and alcohol (ethanol) Extraction.

Products are prepared from herbs or herb blends by extraction with solvent (hot water, 80% ethanol, or ethyl acetate) under reflux for 30-60 minutes, separated by filtration to obtain a filtrate, and air dried for further analysis. The filtrates were combined, diluted or concentrated prior to determination of activities.

### Example 2: combination of OMN54 with Casodex® (bicalutamide) on Prostate Cancer Cells

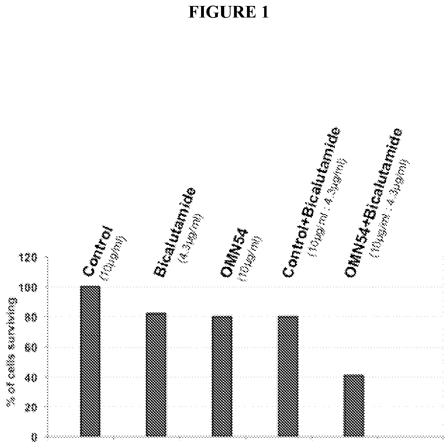

The combination of OMN54 with Casodex® (bicalutamide) achieved greater anti-tumor growth effect than either OMN54 or bicalutamide alone in C4-2 castration-resistant human prostate cancer cells in vitro (Figure 1). Treatment with either bicalutamide (10 µM) or OMN54 (10 µg/ml) alone had minimum inhibitory effect on C4-2 cells, however, the combination of OMN54 (10 µg/ml) with bicalutamide (10 µM) significantly inhibited cell growth at 48 h (see Figure 1).

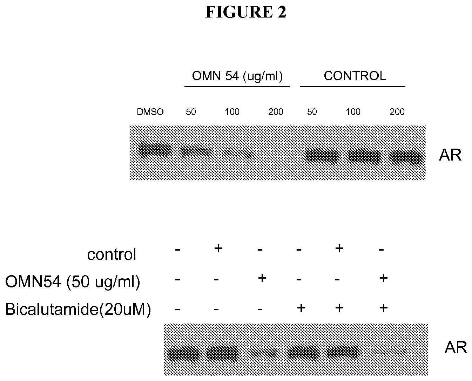

The effects of these two agents and their combination on Androgen Receptor (AR) expression. The expression of AR protein was reduced in the OMN54 treatment group, but not in the control group. The AR expression was further decreased in the combination of OMN54 and Casodex® (bicalutamide) group compared to either the OMN54 or bicalutamide group alone (see Figure 2).

### Example 3: Effect of OMN54 and bicalutamide treatment on AR expression

Figure 2 shows the effect of OMN54 and bicalutamide treatment on AR expression in C4-2 cells by Western blot analysis.

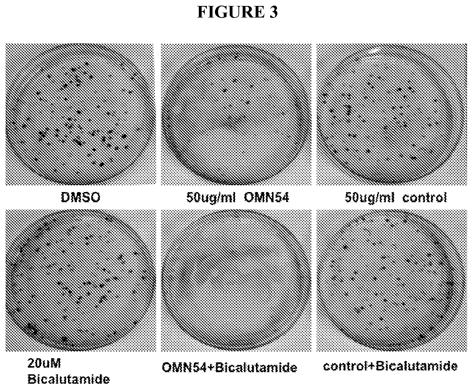

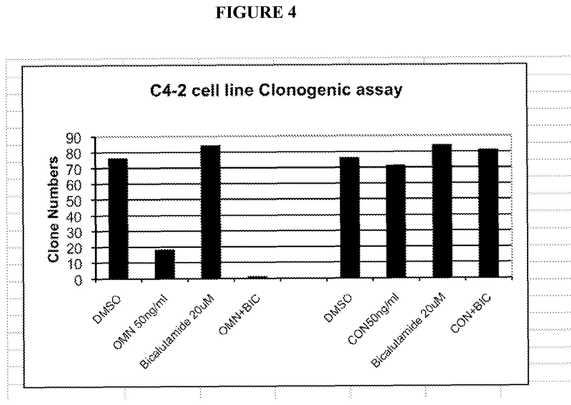

Using a clonogenic assay, it was found that the combination of OMN54 with Casodex® (bicalutamide) achieved greater anti-tumor growth effect than either OMN54 or bicalutamide alone in C4-2 castration-resistant human prostate cancer cells in vitro (Figure 3). In this assay, treatment with bicalutamide (20 µM) demonstrated no effect, while OMN54 (50 µg/ml) demonstrated significant inhibitory effect on C4-2 cells, however, the combination of OMN54 (50 µg/ml) with bicalutamide (20 µM) inhibited cell growth even further at 24 h (see Figure 3 and Figure 4).

### Example 4: Effect of OMN54 and bicalutamide treatment on clonogenic ability

Figure 3 shows the effect of combination of OMN54 and bicalutamide on clonogenic ability in C4-2 cells. C4-2 cells were treated with OMN54 and bicalutamide either alone or together for 24 hr. The cells were then trypsinized and plated at 800 cells per dish and grown for 2 weeks. The cells were fixed and stained. These data suggest that combination of OMN54 and bicalutamide synergistically inhibits castration-resistant prostate tumor growth.

Figure 4 also shows measurement of the effect of combination of OMN54 and bicalutamide on clonogenic ability in C4-2 cells.

The effects of OMN54 with Casodex® (bicalutamide) on androgen-sensitive LNCaP prostate cancer cell growth in vitro. As with previous studies, the combination of OMN54 with bicalutamide demonstrated more effect than either of the agents alone (see Figure 4).

### Example 4: Effect of OMN54 and bicalutamide treatment on LNCaP cell growth

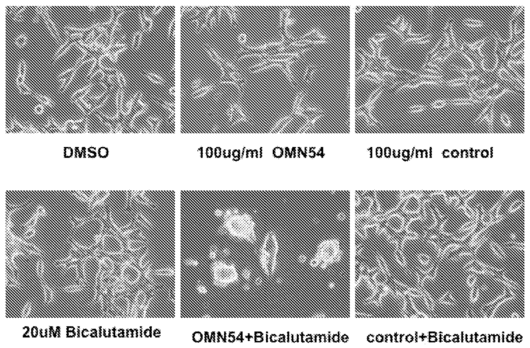

Figure 5 shows the effect of combination of OMN54 and bicalutamide on LNCaP cell growth. LNCaP cells were treated with OMN54 and bicalutamide either alone or combination for 48 hr. These pictures show that combination of OMN54 and bicalutamide induces apoptosis, while OMN54 or bicalutamide alone mostly inhibits cell growth.

This experimental evidence suggests that further inhibition of Androgen Receptor (AR) signaling by combining OMN54 (which reduces AR protein expression) and anti-androgen bicalutamide (which blocks ligand binding to AR or reduces ligand levels) may achieve better anti-cancer efficacy than anti-androgen therapy alone for castration-resistant prostate cancer. Additionally, it ise now demonstrated that the combination of these two agents appears to have greater therapeutic effect on LNCaP androgen-sensitive prostate cancer cells than either agent alone.

### Example 5: Establishment of a human prostate cancer tissue xenograft/mouse model

A more predictive human tumor tissue xenograft model was used to assess efficacy of the botanical compositions. (Clin Cancer Research 2006: 12(13); 4043-4054) Human tumor grafts are better than cell line injections and human tumors grafted on kidney capsule have more immediate blood supply than subcutaneous grafts, thus more biological diversity is retained. The xenograft model more closely resembles clinical cancer than traditional in vivo models

Preclinical testing of prostate cancer therapeutics has been largely carried out using xenograft models in which human prostate cancer cell lines have been subcutaneous ly injected into immunodeficient mice. However, cancer cell xenografts may not accurately mimic the behavior of prostate tumors in vivo. In fact, cancer cell line xenograft models have a poor record of accurately predicting the clinical efficacy of anticancer agents. A novel xenograft model was established for a variety of pre-cancerous and cancerous human tissues, including prostate cancer tissue. Most importantly, the xenografts in the model retain the histological characteristics of the parental tissue. For selected types of cancer that the xenografts respond to therapy in a manner similar to that observed in patients. For example, prostate cancer tissue grown in SCID mice showed a dramatic response to androgen ablation therapy as regularly found in the clinic.

Xenografts of human prostate cancer cell line DU145 were grown in vivo. One of two tissue xenografts grew to the size of a walnut. Tumors are grafted to the renal site survive and retain their original histopathology and differentiation marker profile, even after serial passages. The prostate cancer tissue very rapidly grows in SCID mice with a doubling time about 5 days. Cytogenetic analyses show some abnormal chromosomes. Not only are there translocations, there are also deletions and duplication of chromosomal segments (Note: since each chromosome has its own display color, more than one color along the length of a chromosome indicates a translocation). The Spectral Karyotyping (SKY) analysis shows that the tissue of a DU145 cancer xenograft contained only a low number of karyotypic alterations, although the cancer is highly advanced.

### Example 6: Efficacy study on prostate cancer cell line (DU145)

Tumor xenografts from a prostate cell line DU145 were cut into 2 mm³ pieces and grafted into SCID/nod mice. Treatment was started at day 13 (mean volume = 15.6 mm³). The mice were divided into 3 equal groups for treatment with saline; Ganoderma lucidum, Salvia miltiorrhiza, and Scutellaria barbata at 3.3 IC₅₀; and estramustine sodium phosphate (EMCYT®) and docetaxel (E+D). The combination of Ganoderma lucidum, Salvia miltiorrhiza, and Scutellaria barbata showed a significant inhibitory effect comparable to the E+D regimen. OMN54 significantly inhibited growth of DU145 (androgen-independent) human prostate cancer tumors in vivo. Importantly, this effect on tumor volume was comparable to that of standard chemotherapy which is associated with significant toxicities to patients.

### Example 7: In vivo anti-prostate tumor activity assay

6-week old male nude mice (BALB/c-nu/nu) are used for the experimental animal model. Animals are cut open from the abdomen and inoculated with the human prostate cancer cell lines-LNCaP cells (2x 10⁶ cells/5(^I/Hanks Buffered Saline Solution/mice) from back side of the prostate with a 30g needle. The cut abdomens are then sutured with 5-0 thread. After 2 weeks with regular feeding, blood is drawn from each animal for measuring the serum PSA value. The compositions of the invention are administered orally to the mice as follows; 3 groups of 6 mice received 43.65, 14.4, or 4.3 mg/animal/day for 21 days. Age-matched control mice are treated with saline for the same period.

Grafts are then harvested to determine the effect on tumor volume, histology, apoptosis index (Tunel assay) and proliferation index (proliferation marker Ki67 staining). The TUNEL assay detects apoptosis-induced DNA fragmentation through a quantitative fluorescence assay. Terminal deoxynucleotidyl transferase (TdT) catalyzes the incorporation of bromo-deoxyuridine (BrdU) residues into the fragmenting nuclear DNA at the 3'-hydroxyl ends by nicked end labeling. A TRITC-conjugated anti-BrdU antibody can then label the 3'-hydroxyl ends for detection.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the scope of the appended claims.

## Claims

1. A botanical composition and bicalutamide for use in the treatment or reducing the severity of prostate cancer in a subject, wherein the botanical composition comprises extracts in organic medium of *Ganoderma lucidum, Salvia miltiorrhiza,* and *Scutellaria barbata,* wherein each extract comprises from about 1 to about 90 percent w/w and wherein the botanical composition is effective for reducing androgen receptor protein expression; wherein
an effective amount of the botanical composition and an effective amount of bicalutamide are to be co-administered, and
wherein the co-administration of bicalutamide and the botanical composition achieves more effective therapy than administration of either agent alone.

2. A botanical composition and bicalutamide for use according to claim 1, wherein the bicalutamide is to be administered at 25, or 50 or 150 mg per day.

3. A botanical composition and bicalutamide for use according to claim 1, wherein the co-adminstration is administration of the botanical composition and the administration of bicalutamide is between 0 and 24 hours apart.

4. A botanical composition and bicalutamide for use according to claim 3, wherein the extract is a non-alcoholic organic extract.

5. A botanical composition and bicalutamide for use according to claim 4, wherein the extract is made with ethyl acetate ester.

6. A botanical composition and bicalutamide for use according to claim 1, wherein co-administration of bicalutamide and the botanical composition results in reduced toxicity and side-effects compared to administration of chemical compound alone.

7. A botanical composition and bicalutamide for use according to claim 1, wherein the treatment or reduction of the severity of prostate cancer further comprises administration of a luteinizing hormone releasing hormone (LHRH) to the subject.

8. A botanical composition and bicalutamide for use according to claim 1, wherein bicalutamide and the botanical composition are to be administered in a single dosage form.

9. A botanical composition and bicalutamide for use according to claim 1, wherein bicalutamide and the botanical composition are to be administered in a plurality of dosage forms.

10. A botanical composition and bicalutamide for use according to claim 1, wherein bicalutamide and the botanical composition are to be administered orally.

11. A botanical composition and bicalutamide for use according to claim 1, wherein the prostate cancer is a castration-resistant prostate cancer.

12. A botanical composition and bicalutamide for use according to claim 1, wherein the prostate cancer is an androgen-sensitive prostate cancer.

13. A botanical composition and bicalutamide for use according to claim 1, wherein bicalutamide is to be administered in a sub-therapeutic dose.

14. A tablet dosage form comprising:
a) a first layer comprising a tablet of bicalutamide, wherein the tablet is inlayed in the first layer with one or more other pharmaceutically acceptable excipients; and
b) a second layer comprising a composition comprising organic extracts of *Ganoderma lucidum*, *Scutellaria barbata*, and *Salvia miltiorrhiza,* and optionally other pharmaceutically acceptable excipients.

15. The dosage form of claim 14, comprising a bilayered dosage form.

16. The dosage form of claim 14, wherein the tablet is coated with one or more enteric polymers, pharmaceutically acceptable seal coat polymers or rate controlling polymers.

17. The dosage form of claim 14, wherein the tablet is coated with one or more enteric polymers, pharmaceutically acceptable seal coat polymers or rate controlling polymers.

18. A kit comprising:
a dosage form comprising an effective amount of a botanical composition comprising extracts in organic medium of *Ganoderma lucidum, Salvia miltiorrhiza,* and *Scutellaria barbata,* wherein each extract comprises from about 1 to about 90 percent w/w and wherein the botanical composition is effective for reducing androgen receptor protein expression;
a dosage form comprising an effective amount of bicalutamide; and
optionally one or more pharmaceutically acceptable excipients.

19. The kit of claim 18, wherein the pharmaceutically acceptable excipient comprises one or more of emulsifiers and oil.

20. The kit of claim 19, wherein the emulsifier is selected from one or more of fatty acids, polyoxyethylene glycerol esters of fatty acids, polooxylated castor oil, ethylene glycol esters, propylene glycol esters glyceryl esters of fatty acids, sorbitan esters, polyglyceryl esters, fatty alcohol ethoxylates, ethoxylated propoxylated block copolymers, polyethylene glycol esters of fatty acids, cremophores, glycerol monocaprylate/caprate, Gelucire, Capryol, Captex, Acconon, transcutol, and triacetin.

21. The kit of claim 18, wherein the pharmaceutically acceptable excipient comprises one or more of antioxidants and diluents.

22. The dosage form of claims 14 or 18 that comprises the botanical composition, comprising an effective amount of *Ganoderma lucidum* extract selected from 33%, 35%, 40%, 42%, 44%, 45%, 46%, 46.5%, 47%, 47.5%, 48%, 48.5%, 49%, 49.5% and 50%.

23. The dosage form of claims 14 or 18 that comprises the botanical composition, comprising an effective amount of *Scutellaria barbata* extract selected from 33%, 35%, 40%, 42%, 44%, 45%, 46%, 46.5%, 47%, 47.5%, 48%, 48.5%, 49%, 49.5% and 50%.

24. The dosage form of claims 14 or 18 that comprises the botanical composition, comprising an effective amount of *Salvia miltiorrhiza* extract selected from 1 %, 1.5%, 2 %, 2.5%, 3%, 3.5%, 4%, 4.5%, 5%, 5.5%, 6%, 6.5%, 7%, 7.5%, 8%, 8.5%, 9%, 9.5% and 10%.

25. The kit of claim 18, wherein bicalutamide is provided in a tablet form and the botanical extracts composition is provided in a capsule form.

26. The kit of claim 18, wherein both dosage forms are for oral administration.

27. The kit of claim 18 comprising a single package containing both dosage forms.

## Patentansprüche

1. Pflanzliche Zusammensetzung und Bicalutamid zur Verwendung bei der Behandlung oder Verminderung der Schwere von Prostatakrebs bei einem Individuum, wobei die pflanzliche Zusammensetzung Extrakte von *Ganoderma lucidum, Salvia miltiorrhiza* und *Scutellaria barbata* in organischem Medium umfasst, wobei die Extrakte jeweils etwa 1 bis etwa 90 Gewichtsprozent umfassen und wobei die pflanzliche Zusammensetzung bei der Verringerung der Androgenrezeptorproteinexpression wirksam ist; wobei
eine wirksame Menge der pflanzlichen Zusammensetzung und eine wirksame Menge Bicalutamid gemeinsam zu verabreichen sind und
wobei durch die gemeinsame Verabreichung von Bicalutamid und der pflanzlichen Zusammensetzung eine wirksamere Therapie erzielt wird als durch individuelle Verabreichung der beiden Mittel.

2. Pflanzliche Zusammensetzung und Bicalutamid zur Verwendung nach Anspruch 1, wobei das Bicalutamid zu 25 oder 50 oder 150 mg pro Tag zu verabreichen ist.

3. Pflanzliche Zusammensetzung und Bicalutamid zur Verwendung nach Anspruch 1, wobei bei der gemeinsamen Verabreichung die Verabreichung der pflanzlichen Zusammensetzung und die Verabreichung von Bicalutamid in einem Abstand zwischen 0 und 24 Stunden erfolgen.

4. Pflanzliche Zusammensetzung und Bicalutamid zur Verwendung nach Anspruch 3, wobei es sich bei dem Extrakt um einen nichtalkoholischen organischen Extrakt handelt.

5. Pflanzliche Zusammensetzung und Bicalutamid zur Verwendung nach Anspruch 4, wobei der Extrakt mit Essigsäureethylester hergestellt wird.

6. Pflanzliche Zusammensetzung und Bicalutamid zur Verwendung nach Anspruch 1, wobei gemeinsame Verabreichung von Bicalutamid und der pflanzlichen Zusammensetzung zu einer Verringerung von Toxizität und Nebenwirkungen verglichen mit Verabreichung einer chemischen Verbindung allein führt.

7. Pflanzliche Zusammensetzung und Bicalutamid zur Verwendung nach Anspruch 1, wobei die Behandlung oder Verminderung der Schwere von Prostatakrebs ferner Verabreichung eines LHRH (Luteinizing Hormone Releasing Hormone) an das Individuum umfasst.

8. Pflanzliche Zusammensetzung und Bicalutamid zur Verwendung nach Anspruch 1, wobei Bicalutamid und die pflanzliche Zusammensetzung in einer einzelnen Darreichungsform zu verabreichen sind.

9. Pflanzliche Zusammensetzung und Bicalutamid zur Verwendung nach Anspruch 1, wobei Bicalutamid und die pflanzliche Zusammensetzung in mehreren Darreichungsformen zu verabreichen sind.

10. Pflanzliche Zusammensetzung und Bicalutamid zur Verwendung nach Anspruch 1, wobei Bicalutamid und die pflanzliche Zusammensetzung oral zu verabreichen sind.

11. Pflanzliche Zusammensetzung und Bicalutamid zur Verwendung nach Anspruch 1, wobei es sich bei dem Prostatakrebs um ein kastrationsresistentes Prostatakarzinom handelt.

12. Pflanzliche Zusammensetzung und Bicalutamid zur Verwendung nach Anspruch 1, wobei es sich bei dem Prostatakrebs um ein androgensensitives Prostatakarzinom handelt.

13. Pflanzliche Zusammensetzung und Bicalutamid zur Verwendung nach Anspruch 1, wobei Bicalutamid in einer subtherapeutischen Dosis zu verabreichen ist.

14. Tablettendarreichungsform, umfassend:
a) eine erste Schicht, die eine Bicalutamid-Tablette umfasst, wobei die Tablette als Inlay in der ersten Schicht mit einem oder mehreren anderen pharmazeutisch unbedenklichen Hilfsstoffen vorliegt; und
b) eine zweite Schicht, die eine organische Extrakte von *Ganoderma lucidum, Scutellaria barbata* und *Salvia miltiorrhiza* umfassende Zusammensetzung und gegebenenfalls andere pharmazeutisch unbedenkliche Hilfsstoffe umfasst.

15. Darreichungsform gemäß Anspruch 14, umfassend eine zweischichtige Darreichungsform.

16. Darreichungsform gemäß Anspruch 14, wobei die Tablette mit einem oder mehreren magensaftresistenten Polymeren, pharmazeutisch unbedenklichen "Seal coat"-Polymeren oder geschwindigkeitsbestimmenden Polymeren beschichtet ist.

17. Darreichungsform gemäß Anspruch 14, wobei die Tablette mit einem oder mehreren magensaftresistenten Polymeren, pharmazeutisch unbedenklichen "Seal coat"-Polymeren oder geschwindigkeitsbestimmenden Polymeren beschichtet ist.

18. Kit, umfassend:
eine Darreichungsform, die eine wirksame Menge einer pflanzlichen Zusammensetzung umfasst, welche Extrakte von *Ganoderma lucidum, Salvia miltiorrhiza* und *Scutellaria barbata* in organischem Medium umfasst, wobei die Extrakte jeweils etwa 1 bis etwa 90 Gewichtsprozent umfassen und wobei die pflanzliche Zusammensetzung bei der Verringerung der Androgenrezeptorproteinexpression wirksam ist;
eine Darreichungsform, die eine wirksame Menge Bicalutamid umfasst; und
gegebenenfalls einen oder mehrere pharmazeutisch unbedenkliche Hilfsstoffe.

19. Kit gemäß Anspruch 18, wobei der pharmazeutisch unbedenkliche Hilfsstoff einen oder mehrere Emulgatoren und Öl umfasst.

20. Kit gemäß Anspruch 19, wobei der Emulgator aus einem oder mehreren von Fettsäuren, Polyoxyethylenglycerinestern von Fettsäuren, polooxyliertem Ricinusöl, Ethylenglykolestern, Propylenglykolestern, Glycerylestern von Fettsäuren, Sorbitanestern, Polyglycerylestern, Fettalkoholethoxylaten, ethoxylierten propoxylierten Block-Copolymeren, Polyethylenglykolestern von Fettsäuren, Cremophoren, Glycerinmonocaprylat/-caprat, Gelucire, Capryol, Captex, Acconon, Transcutol und Triacetin ausgewählt ist.

21. Kit gemäß Anspruch 18, wobei der pharmazeutisch unbedenkliche Hilfsstoff ein oder mehrere Antioxidantien und Verdünnungsmittel umfasst.

22. Darreichungsform gemäß Anspruch 14 oder 18, die die pflanzliche Zusammensetzung umfasst, umfassend eine wirksame Menge *Ganoderma lucidum*-Extrakt ausgewählt aus 33%, 35%, 40%, 42%, 44%, 45%, 46%, 46,5%, 47%, 47,5%, 48%, 48,5%, 49%, 49,5% und 50%.

23. Darreichungsform gemäß Anspruch 14 oder 18, die die pflanzliche Zusammensetzung umfasst, umfassend eine wirksame Menge *Scutellaria* barbata-Extrakt ausgewählt aus 33%, 35%, 40%, 42%, 44%, 45%, 46%, 46,5%, 47%, 47,5%, 48%, 48,5%, 49%, 49,5% und 50%.

24. Darreichungsform gemäß Anspruch 14 oder 18, die die pflanzliche Zusammensetzung umfasst, umfassend eine wirksame Menge *Salvia miltiorrhiza*-Extrakt ausgewählt aus 1%, 1,5%, 2%, 2,5%, 3%, 3,5%, 4%, 4,5%, 5%, 5,5%, 6%, 6,5%, 7%, 7,5%, 8%, 8,5%, 9%, 9,5% und 10%.

25. Kit gemäß Anspruch 18, wobei Bicalutamid in Tablettenform und die Pflanzliche-Extrakte-Zusammensetzung in Kapselform bereitgestellt wird.

26. Kit gemäß Anspruch 18, wobei beide Darreichungsformen zur oralen Verabreichung vorgesehen sind.

27. Kit gemäß Anspruch 18, umfassend eine Einzelpackung, die beide Darreichungsformen enthält.

## Revendications

1. Composition botanique et bicalutamide, pour une utilisation dans le traitement ou la réduction de la gravité du cancer de la prostate chez un sujet, où la composition botanique comprend des extraits dans un milieu organique de *Ganoderma lucidum, Salvia miltiorrhiza,* et *Scutellaria barbata,* où chaque extrait constitue d'environ 1 à environ 90% p/p et où la composition botanique est efficace pour la réduction de l'expression de la protéine de récepteurs d'androgènes ; où
une quantité efficace de la composition botanique et une quantité efficace de bicalutamide sont destinées à être coadministrées, et
où la co-administration de bicalutamide et de la composition botanique conduit à une thérapie plus efficace que l'administration de l'un ou l'autre agent seul.

2. Composition botanique et bicalutamide pour une utilisation selon la revendication 1, où le bicalutamide est destiné à être administré à 25, ou 50 ou 150 mg par jour.

3. Composition botanique et bicalutamide pour une utilisation selon la revendication 1, où la co-administration est l'administration de la composition botanique et l'administration de bicalutamide est effectuée entre 0 et 24 heures d'intervalle.

4. Composition botanique et bicalutamide pour une utilisation selon la revendication 3, où l'extrait est un extrait organique non alcoolique.

5. Composition botanique et bicalutamide pour une utilisation selon la revendication 4, où l'extrait est préparé avec un ester d'acétate d'éthyle.

6. Composition botanique et bicalutamide pour une utilisation selon la revendication 1, où la co-administration de bicalutamide et de la composition botanique entraîne une toxicité et des effets secondaires réduits par rapport à l'administration du composé chimique seul.

7. Composition botanique et bicalutamide pour une utilisation selon la revendication 1, où le traitement ou la réduction de la gravité du cancer de la prostate comprend en outre l'administration d'une hormone de libération des gonadotrophines (LHRH) au sujet.

8. Composition botanique et bicalutamide pour une utilisation selon la revendication 1, où le bicalutamide et la composition botanique sont destinés à être administrés dans une forme de dosage unique.

9. Composition botanique et bicalutamide pour une utilisation selon la revendication 1, où le bicalutamide et la composition botanique sont destinés à être administrés dans une pluralité de formes de dosage.

10. Composition botanique et bicalutamide pour une utilisation selon la revendication 1, où le bicalutamide et la composition botanique sont destinés à être administrés par voie orale.

11. Composition botanique et bicalutamide pour une utilisation selon la revendication 1, où le cancer de la prostate est un cancer de la prostate résistant à la castration.

12. Composition botanique et bicalutamide pour une utilisation selon la revendication 1, où le cancer de la prostate est un cancer de la prostate sensible aux androgènes.

13. Composition botanique et bicalutamide pour une utilisation selon la revendication 1, où le bicalutamide est destiné à être administré selon une dose sub-thérapeutique.

14. Forme de dosage en comprimés, comprenant :
a) une première couche comprenant un comprimé de bicalutamide, où le comprimé est incrusté dans la première couche avec un ou plusieurs autres excipients pharmaceutiquement acceptables ; et
b) une deuxième couche comprenant une composition comprenant des extraits organiques de *Ganoderma lucidum, Scutellaria barbata,* et *Salvia miltiorrhiza,* et éventuellement d'autres excipients pharmaceutiquement acceptables.

15. Forme de dosage selon la revendication 14, comprenant une forme de dosage bicouche.

16. Forme de dosage selon la revendication 14, dans laquelle le comprimé est revêtu par un ou plusieurs polymères entériques, des polymères de couche de scellement pharmaceutiquement acceptables ou des polymères de contrôle cinétique.

17. Forme de dosage selon la revendication 14, dans laquelle le comprimé est revêtu par un ou plusieurs polymères entériques, des polymères de couche de scellement pharmaceutiquement acceptables ou des polymères de contrôle cinétique.

18. Kit, comprenant :
une forme de dosage comprenant une quantité efficace d'une composition botanique comprenant des extraits dans un milieu organique de *Ganoderma lucidum, Salvia miltiorrhiza,* et *Scutellaria barbata,* où chaque extrait constitue d'environ 1 à environ 90% p/p et où la composition botanique est efficace pour la réduction de l'expression de la protéine de récepteurs d'androgènes ;
une forme de dosage comprenant une quantité efficace de bicalutamide ; et
éventuellement un ou plusieurs excipients pharmaceutiquement acceptables.

19. Kit selon la revendication 18, dans lequel l'excipient pharmaceutiquement acceptable comprend un ou plusieurs parmi des émulsifiants et de l'huile.

20. Kit selon la revendication 19, dans lequel l'émulsifiant est choisi parmi un ou plusieurs parmi les acides gras, les esters d'acides gras et de glycérol polyoxyéthylénés, l'huile de ricin polyoxyle, les esters d'éthylène glycol, les esters de propylène glycol, les esters de glycérol et d'acides gras, les esters de sorbitane, les esters de polyglycérol, les éthoxylates d'alcool gras, les copolymères blocs éthoxylés et propoxylés, les esters d'acides gras et de polyéthylène glycol, les Cremophor, le monocaprylate/caprate de glycérol, le Gelucire, le Capryol, le Captex, l'Acconon, le Transcutol, et la triacétine.

21. Kit selon la revendication 18, dans lequel l'excipient pharmaceutiquement acceptable comprend un ou plusieurs antioxydants et diluants.

22. Forme de dosage selon les revendications 14 ou 18, comprenant la composition botanique, comprenant une quantité efficace d'extrait de *Ganoderma lucidum* choisie parmi 33%, 35%, 40%, 42%, 44%, 45%, 46%, 46,5%, 47%, 47,5%, 48%, 48,5%, 49%, 49,5%, et 50%.

23. Forme de dosage selon les revendications 14 ou 18, comprenant la composition botanique, comprenant une quantité efficace d'extrait de *Scutellaria barbata* choisie parmi 33%, 35%, 40%, 42%, 44%, 45%, 46%, 46,5%, 47%, 47,5%, 48%, 48,5%, 49%, 49,5%, et 50%.

24. Forme de dosage selon les revendications 14 ou 18, comprenant la composition botanique, comprenant une quantité efficace d'extrait de *Salvia miltiorrhiza* choisie parmi 1%, 1,5%, 2%, 2,5%, 3%, 3,5%, 4%, 4,5%, 5%, 5,5%, 6%, 6,5%, 7%, 7,5%, 8%, 8,5%, 9%, 9,5%, et 10%.

25. Kit selon la revendication 18, dans lequel le bicalutamide est fourni sous forme de comprimé et la composition d'extraits botaniques est fournie sous forme de capsule.

26. Kit selon la revendication 18, dans lequel les deux formes de dosage sont destinées à une administration orale.

27. Kit selon la revendication 18, comprenant un emballage unique contenant les deux formes de dosage.
